# EUROPÄISCHE PATENTSCHRIFT

(11) **EP 2 959 855 B1**
(45) Veröffentlichungstag und Bekanntmachung des Hinweises auf die Patenterteilung: **10.04.2019**
(21) Anmeldenummer: 15173830.9
(22) Anmeldetag: 25.06.2015
(51) Int. Cl.: A61B 18/00, A61B 18/14

(54) **ELEKTROCHIRURGISCHE RESEKTIONSSCHLINGE MIT EINEM FREIGELEGTEN TEIL UND MIT EINEM MIT EINER ISOLATION VERSEHENEN TEIL**
ELECTROSURGICAL RESECTION LOOP WITH AN EXPOSED PART AND WITH A PART PROVIDED WITH AN INSULATION
BOUCLE DE RÉSECTION ÉLECTROCHIRURGICALE AVEC UNE PARTIE EXPOSÉE ET AVEC UNE PARTIE POURVUE D'UNE ISOLATION

(30) Priorität: 25.06.2014 EP 14173891
(43) Veröffentlichungstag der Anmeldung: 30.12.2015
(73) Patentinhaber: Farin, Günter, 72070 Tübingen (DE); endox Feinwerktechnik GmbH, 72574 Bad Urach (DE)
(72) Erfinder: FARIN, Günter, 72070 Tübingen (DE); HERNIK, Matthias, 72581 Dettingen/Erms (DE)
(74) Vertreter: Lohr, Jöstingmeier & Partner

(56) Entgegenhaltungen:
- WO-A1-2013/064576
- DE-A1- 3 220 940
- DE-U1-202010 008 674
- US-A- 5 919 190
- US-A1- 2011 166 565
- US-A1- 2011 166 569

## Beschreibung

### Technisches Gebiet

Die Erfindung betrifft eine hochfrequenzchirurgische (HF-chirurgische) Resektionsschlinge für Instrumente oder als Teil von Instrumenten zum endoskopisch kontrollierten Entfernen polypenartig oder flach wachsender pathologischer Mukosa-Areale (Läsionen) des Magendarmtrakts. Diese Instrumente werden u.a. Polypektom oder - pars pro toto - Polypenschlingen genannt.

### Stand der Technik

Eines der ersten dieser Instrumente wird im Jahre 1971 im Deutschen Gebrauchsmuster Nr. 71115781 beschrieben. Heute wie bereits damals bestehen diese Instrumente im Wesentlichen aus einem flexiblen ca. 2 m langen Katheter aus einem Kunststoff, einem in dem Katheter beweglichen metallischen Manipulationsdraht, einer HF-chirurgischen metallischen Resektionsschlinge an einem - dem distalen - Ende des Manipulationsdrahts und einem Manipulationsgriff am anderen - dem proximalen - Ende des Manipulationsdrahts und Katheters. Der Katheter mit der Resektionsschlinge dieser Instrumente werden seit mehr als 40 Jahren durch Instrumentier- bzw. Arbeitskanäle flexibler Endoskope angewendet um hiermit Läsionen der Mukosa des Magendarmtrakts als Prophylaxe gegen Magen-Darm-Krebs HF-chirurgisch zu entfernen.

Wurden mit diesen Instrumenten bis vor ca. 20 Jahren nur relativ kleine Läsionen (< 2 cm) entfernt, so besteht heute der Trend, auch große Läsionen (> 2 cm) hiermit zu entfernen. Allerdings stehen diesem Trend einige Probleme entgegen. Je größer die Läsion, desto größer ist auch die Wahrscheinlichkeit eines manifesten Karzinoms in dieser Läsion. Deswegen fordern Onkologen, dass insbesondere große Läsionen in sano, also bis ins angrenzende gesunde Gewebe entfernt werden. Um pathohistologisch sicher prüfen zu können, ob die Entfernung der Läsion wirklich in sano erfolgte und ob in der entfernten Läsionen bereits ein Karzinom (Krebsgewebe) vorhanden ist, und wenn ja, ob dieses Karzinom bereits in Lymph- und/oder arterielle Gefäße infiltriert ist, also bereits ein Risiko von Metastasen in anderen Organen besteht, fordern die Pathologen die Entfernung der Läsion en bloc, also in einem Stück und inklusive der unter der Läsion befindlichen Submukosa, zumindest des oberen Drittels (die sm1) der Submukosa. Um diese Forderung der Pathologen zu erfüllen, ist es zweckmäßig, beispielsweise physiologische NaCI-Lösung in die Submukosa zu injizieren, so dass diese infolge Absorption der NaCl Lösung quillt, also dicker wird. Allerdings wird hierdurch auch die spezifische elektrische Leitfähigkeit der Submukosa größer, was insbesondere während der Anschnittphase bei der HF-chirurgischen Resektion die Anschnittverzögerung nachteilig verlängert. Da mit Rücksicht auf die o.g. Forderungen der Onkologen Läsionen mit einem zirkumferenten Sicherheitsabstand von ca. 5 mm entfernt werden müssen, muss beispielsweise bei einer 3 cm großen Läsion ein Mukosa-Areal mit 4 cm Durchmesser en bloc HF-chirurgisch entfernt werden.

Die HF-chirurgische en-bloc Entfernung großer Läsionen (> 2 cm) mit HF-chirurgischen Schlingen bzw. Polypektomieschlingen ist u.a. insofern problematisch, als für einen HF-chirurgischen Schnitt während der Anschnittphase, also in der Zeit zwischen Aktivierung des HF-Strom-Generators und der zum HF-chirurgischen Schneiden von Geweben erforderlichen elektrischen Lichtbogen zwischen Polypektomieschlinge und Gewebe ein HF-Strom von mindestens 0,5 A_{eff} pro cm HF-chirurgisch aktiver Schlingenlänge erforderlich ist, damit der Schnitt mit möglichst geringer Verzögerung, der sog. Anschnittverzögerung, entstehen kann. Bei zu langer Anschnittverzögerung besteht das Risiko, dass die Organwand unterhalb der Läsion thermisch geschädigt wird und postoperativ eine post Polypektomie Perforation der Organwand entstehen kann. Da in der Endoskopie verfügbare HF-Generatoren einen HF-Strom mit maximal nur 1,5 bis 2,0 A_{eff} liefern, können hiermit nur Läsionen bei einer effektiven Schlingenlänge von 3 bis 4 cm, was einem Durchmesser von ca. 1 bis 1,5 cm entspricht, ausreichend verzögerungsfrei entfernt werden, mit der Konsequenz, dass insbesondere große Läsionen (> 2 cm) nicht komplett en bloc entfernt werden können. Deswegen werden große Läsionen entweder mit bisher verfügbaren Polypektomieschlingen in mehreren kleineren Stücken (piecemeal technique) oder mit dem in Japan entwickelten Verfahren der Endoskopischen Submukosa Dissektion (ESD) entfernt. Die piecemeal technique erfüllt nicht die o.g. Forderungen der Pathologen, Läsionen en bloc zu entfernen. Die ESD ist schwierig und sehr zeitaufwendig in der Anwendung.

Das Dokument DE 20 2010 008674 U1 beschreibt eine HF-chirurgische Resektionsschlinge.

In der Deutschen Offenlegungsschrift DE 100 28 413 A1 werden elektrochirurgische Instrumente mit jeweils einer Elektrode, u.a. auch Schlingenelektroden, mit einem Elektrodenkern und einer den Elektrodenkern partiell abdeckenden Isolierummantelung offenbart. Dabei ist an der Schlingenspitze die freie Elektrodenfläche des Elektrodenkerns symmetrisch durch eine Isolierummantelung mit einer Vielzahl von Öffnungen bzw. kleinen Löchern oder eine den Elektrodenkern nur in einem Kreissegment umschließende Isolierummantelung reduziert.

Hierdurch soll die zum Schneiden erforderliche Stromstärke reduziert werden.

Mit diesen Schlingenelektroden kann theoretisch zwar das o.g. Problem einer zu langen Anschnittverzögerung beseitigt werden, die Herstellung dieser Schlingenelektroden ist jedoch sehr aufwendig und folglich teuer.

Ein weiteres Problem bei Anwendung der bisher verfügbaren Polypektomieschlingen besteht darin, das weder der Endoskopiker noch seine Assistenz (z.B. Endoskopieschwester) insbesondere während der HF-chirurgischen Schnittführung beobachten bzw. visuell kontrollieren kann, ob, und wenn ja, wie schnell die HF-chirurgische Resektionsschlinge schließt. Infolge der mehr oder weniger großen Elastizität des langen Katheters einerseits und Manipulationsdrahtes andererseits, sowie der beachtlichen Reibung zwischen Katheter und Manipulationsdraht, und auch infolge des bei bisher verfügbaren Polypektomen großen axialen Totweges (Hysterese) zwischen Manipulationsgriff und Resektionsschlinge, ist eine zuverlässige Kontrolle des Schließens der Schlinge am Manipulationsgriff in der Regel nicht möglich. Insbesondere bei großen Läsionen (> ca. 2 cm) kann weder der Endoskopiker noch die Assistenz sicher kontrollieren, ob die Schlinge nach Aktivieren des HF-Strom Generators schneidet oder nicht schneidet, und wenn es schneidet, wie schnell es schneidet. Eine Kontrolle des Schließens der Schlinge sowie der Geschwindigkeit der Schnittführung ist aber bezüglich der Schnittqualität und insbesondere bezüglich eines schnittsynchronen Gefäßverschlusses sehr wichtig.

### Darstellung der Erfindung

Es ist Aufgabe der Erfindung, eine HF-chirurgische Resektionsschlinge für Instrumente oder als Teil von Instrumenten zum endoskopisch kontrollierten Entfernen polypenartig oder flach wachsender pathologischer Mukosa-Areale (Läsionen) des Magendarmtrakts zu entwickeln, bei welchen die o.g. Probleme nicht vorhanden sind.

Insbesondere ist es Aufgabe der Erfindung, derartige Resektionsschlingen zu entwickeln, bei deren Anwendung die Anschnittverzögerung möglich kurz ist, deren Eigenschaften während der HF-chirurgischen Schnittführung nicht durch die für den HF-chirurgischen Schnitt erforderlichen sehr heißen elektrischen Lichtbogen verändert werden und die trotzdem möglichst einfach und mit geringem Aufwand hergestellt werden können.

Es ist darüber hinaus Aufgabe der Erfindung, eine HF-chirurgische Resektionsschlinge insbesondere für die flexible Endoskopie zu entwickeln, bei welchen der Endoskopiker und/oder die Assistenz (z.B. Endoskopieschwester) das Öffnen sowie das Schließen der Schlinge auch dann visuell kontrollieren bzw. beobachten können, wenn die Schlinge um eine Läsion geschlungen nicht sichtbar ist, was insbesondere während der Schnittführung sehr nachteilig ist.

Die Aufgabe der Erfindung wird mit einer HF-chirurgischen Resektionsschlinge nach dem unabhängigen Patentanspruch 1 gelöst. Weitere Ausführungsformen sind in den abhängigen Patentansprüchen beschrieben.

Die HF-chirurgische Resektionsschlinge umfasst einen ersten Schlingenteil sowie einen zweiten Schlingenteil. Die beiden Schlingenteile umfassen metallischen Draht, vorzugsweise einen Volldraht, besonders bevorzugt aus Stahl oder Nitinol, und sie haben jeweils ein proximales und ein distales Ende. Sie bilden mit ihren distalen Enden eine Schlingenspitze. Dabei können wahlweise zwei Schlingenteile an ihren distalen Enden zu einer Schlingenspitze miteinander verbunden sein. Alternativ kann die Schlinge auch einstückig ausgeführt sein, wobei die beiden Schlingenteile aus einem Teil bestehen. An den proximalen Enden sind die beiden Schlingenteile mit einem Manipulationsdraht verbunden, mit dem sie in einen Katheter hineingezogen und/oder aus diesem herausgeschoben werden können. Weiterhin ist im ersten Schlingenteil der Draht vollständig mit einer elektrischen Isolation versehen, während im zweiten Schlingenteil der Draht in einem proximalen Bereich mit einer elektrischen Isolation versehen und in einem distalen Bereich (zur Schlingenspitze hin) ohne elektrische Isolation ist.

Die elektrische Isolation umschließt den Draht der Schlingenteile vollständig, also in radialer Richtung betrachtet in einem Winkelbereich von 360°, und kann eine Isolationsbeschichtung und/oder eine Ummantelung und/oder ein Schlauch, vorzugsweise ein Schrumpfschlauch, sein.

Eine Vielzahl von Versuchsreihen haben gezeigt, dass erfindungsgemäße Resektionsschlingen, bei welchen nur ein kurzer Teil des Schneiddrahtes ohne elektrische Isolation ist, im Vergleich zu Schlingen aus dem o.g. Stand der Technik ihre HF-chirurgischen Eigenschaften auch während längerer HFchirurgischer Schnitte und sogar bei mehrmaliger Anwendung kaum verändern, während Schlingen nach dem o.g. Stand der Technik ihre beabsichtigten HF-chirurgischen Eigenschaften bereits während der ersten Anwendung durch die heißen elektrischen Lichtbogen, welche deren durchlöcherte oder partielle Isolierung schmilzt oder gar wegbrennt, schnell verändern oder gar verlieren. Erfindungsgemäße Resektionsschlingen können wesentlich besser den hohen Temperaturen elektrischer Lichtbogen, die beim HF-chirurgischen Schneiden entstehenden, widerstehen.

Messungen bei Anwendung verschiedener HF-Chirurgie Generatoren haben gezeigt, dass Schlingen entsprechend der Erfindung auch bei großen Läsionen eine wesentlich kürzere oder gar vernachlässigbar kurze Anschnittverzögerung zeigen. Da in der Endoskopie vorwiegend HF-Strom Generatoren verfügbar sind, die 1,5 Aeff HF-Strom liefern können, und weil für einen weitgehend verzögerungsfreien Anschnitt ein HF-Strom von mindestens ca. 0,5 Aeff erforderlich ist, ist der Schlingenabschnitt ohne elektrische Isolation erfindungsgemäßer Schlingen, der sog. HF-chirurgische Schneiddraht, vorzugsweise nur 1 bis 3 cm lang.

Während des Schneidvorganges wird die Schlinge mithilfe des Manipulationsdrahtes in den Katheter hineingezogen, so dass sich die Schlinge schließt. In der Praxis wird die Schlinge, die in das Gewebe einschneidet immer durch das bereits abgeschnittene Gewebe verdeckt, so dass der Schneidfortschritt endoskopisch nicht sichtbar und folglich auch nicht kontrollierbar ist. Um den Schneidfortschritt endoskopisch sichtbar und kontrollierbar zu machen, weist wenigstens einer/s der Schlingenteile und/oder der Manipulationsdraht wenigstens eine Markierung auf. Eine solche Markierung umfasst bevorzugt abwechselnd schwarze und weiße oder verschieden farbige Ringe. So könnten beispielsweise schwarze Ringe auf einer farbigen (beispielsweise gelb, grün, blau, rot) elektrischen Isolation angebracht sein. Bevorzugt haben die beispielsweise schwarzen Ringe eine Länge von 0,3 bis 1 cm und einen Abstand untereinander von ebenfalls 0,3 bis 1 cm. Selbstverständlich können die Längen und Abstände beliebig gestaltet werden. Anstelle von schwarzen Ringen kann auch eine andere Farbe der Ringe gewählt werden, die sich bevorzugt deutlich von der Farbe der elektrischen Isolation abhebt. Es können auch unterschiedliche Farben der Ringe auf der Isolation einer Schlinge gewählt werden, beispielsweise um bestimmte Öffnungsweiten der Schlinge zu markieren. So könnten zwischen jeweils vier schwarzen Ringen ein fünfter roter Ring angeordnet sein. Es könnte auch durch unterschiedliche Ringe bzw. durch unterschiedliche Abstände der Ringe, beispielsweise nach Art eines Barcodes eine Information über die Position bzw. die Öffnungsweite der Schlinge gegeben werden. Bevorzugt umfasst der Katheter hierfür ein transparentes Material, so dass die Markierungen auch im distalen Ende des Katheters endoskopisch gesehen werden können. Weiterhin könnte auch der Katheter farbige Markierungen aufweisen, die insbesondere in Verbindung mit den Markierungen auf der Schlinge weitere Informationen zur Verfügung stellen. So könnten beispielsweise durch Interferenzen mehrerer Muster präzisere oder auch Positionsangaben eines Schlingenteils im Katheter dargestellt werden. Die hier offenbarten Markierungen sind nicht nur auf Schlingen mit teilweiser Isolation anwendbar. Vielmehr können Sie bei allen Arten von HF-chirurgischen Schlingen eingesetzt werden, die in einen Katheter hineingezogen und/oder aus diesem herausgeschoben werden können.

Vorteilhafterweise kann der Schlingenabschnitt ohne elektrische Isolation mit einer Antirutschbeschichtung oder kleinen, vorzugsweise zentripetal ausgerichteten Zähnen ausgestattet werden, was die Applikation auf bzw. um glitschige Läsionen vereinfacht und das Abrutschen der Schlinge von glitschiger Schleimhaut ver- oder zumindest behindert.

Vorteilhafterweise können alle oben aufgeführten erfindungsgemäßen Schlingen mit einem Manipulationsgriff entsprechend WO 2013/064576 ausgestattet werden oder sein.

### Beschreibung der Zeichnungen

Figur 1 zeigt eine erfindungsgemäße Schlinge in geöffnetem Zustand.
Figur 2 zeigt eine erfindungsgemäße Schlinge in teilweise geschlossenem Zustand.

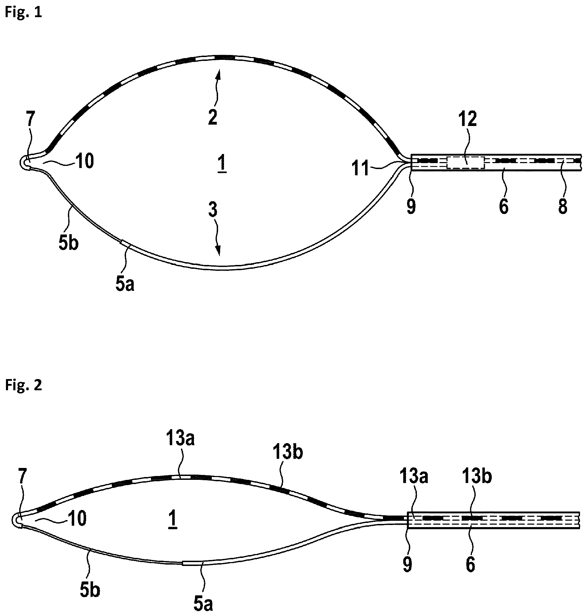

In der Figur 1 ist eine erfindungsgemäße HF-chirurgische Resektionsschlinge 1 für die flexible Endoskopie dargestellt. Sie umfasst ein erstes Schlingenteil 2 sowie ein zweites Schlingenteil 3 aus metallischem Rund- und/oder Flachdraht. Diese Schlingenteile können auch metallische Litze umfassen. Diese Schlingenteile haben jeweils ein proximales Ende 11 und ein distales Ende 10. Die proximalen Enden der Schlingenteile bilden zusammen das proximale Ende 11 der Schlinge und sind mechanisch und elektrisch leitfähig, beispielsweise mittels eines Verbindungselements 12, mit einem Manipulationsdraht 8 in einem Katheter 6 verbunden. Bevorzugt ist der Manipulationsdraht wesentlich steifer als der Schlingendraht.

Die distalen Enden der Schlingenteile 2 und 3 bilden zusammen das distale Ende 10 der Schlinge 1, das als Schlingennase bzw. Schlingenspitze 7 gestaltet sein kann. Die so gestaltete Schlingenspitze wird benötigt, um die Schlinge komplett in das distale Ende 9 des Katheters 6 hineinziehen und aus diesem wieder herausschieben zu können.

Im ersten Schlingenteil 2 ist der Draht vollständig mit einer elektrischen Isolation versehen. Im zweiten Schlingenteil 3 ist der Draht in einem proximalen Bereich 5a mit einer elektrischen Isolation versehen und in einem distalen Bereich 5b ohne elektrische Isolation. Der freiliegende Bereich hat vorzugsweise eine Länge von 1 cm bis 3 cm.

Zur endoskopischen Kontrolle des Öffnens und Schließens der Resektionsschlinge 1 sind Markierungen 13a, 13b auf mindestens einem der Schlingenteile 2 und/oder 3 und / oder auf dem Manipulationsdraht 8 vorgesehen. Bevorzugt bilden diese gegeneinander einen deutlichen visuellen schwarz-weiss Kontrast oder Farbkontrast, so dass diese Markierungen durch einen transparenten Katheter endoskopisch sichtbar sind. Auf diese Weise können axiale Bewegungen des Manipulationsdrahts sowie der markierten Schlingenteile innerhalb eines transparenten Katheters endoskopisch beobachtet und kontrolliert werden. Die Figur 2 zeigt eine teilweise in das distale Ende 9 des Katheters 6 hineingezogene, der Erfindung entsprechende, Schlinge. Die Markierungen 13a und 13b sind durch den Katheter 6 sichtbar. Der Katheter 6 umfasst hierzu ein transparentes Material.

Auf diese Weise können sowohl der Endoskopiker als auch die Assistenten auf einem Video-Monitor beobachten und kontrollieren, ob, und wenn ja, wie schnell sich der Manipulationsdraht und / oder die markierten Schlingeteile im Katheter 6 in axialer Richtung bewegen. Auf diese Weise kann auch die Schnittgeschwindigkeit beurteilt werden, die bekanntlich einen großen Einfluss auf die Schnittqualität, insbesondere auf unbeabsichtigte thermische Schädigungen der resezierten Läsion sowie der unterhalb der resezierten Läsion befindlichen Organwand hat. Außerdem hat die Schnittgeschwindigkeit auch einen großen Einfluss auf den in der Regel beabsichtigten schnittsynchronen Verschluss der durchschnittenen Blutgefäße.

### Bezugszeichenliste

- 1: Resektionsschlinge
- 2: Erster Schlingenteil
- 3: Zweiter Schlingenteil
- 5a: Bereich ohne elektrische Isolation
- 5b: Bereich mit elektrischer Isolation
- 6: Katheter
- 7: Katheterspitze
- 8: Manipulationsdraht
- 9: Distales Katheterende
- 10: distales Ende der Resektionsschlinge
- 11: proximales Ende der Resektionsschlinge
- 12: Verbindungselement
- 13a, 13b: Kontrastierende Markierungen

## Patentansprüche

1. HF-chirurgische Resektionsschlinge (1), umfassend eine Schlinge bestehend aus einem ersten Schlingenteil (2) und einem zweiten Schlingenteil (3), die an ihrem distalen Ende (10) eine Schlingenspitze (7) bilden,
wobei die beiden Schlingenteile metallischen Draht umfassen und wobei im ersten Schlingenteil der Draht komplett mit einer elektrischen Isolation versehen ist,
wobei die Schlinge einstückig ausgeführt ist,
**dadurch gekennzeichnet, dass**
der erste Schlingenteil (2) und der zweite Schlingenteil (3), an ihrem proximalen Ende (11) mit einem in einem Katheter (6) geführten Manipulationsdraht (8) verbunden sind, so dass diese Schlinge mittels dieses Manipulationsdrahts in den Katheter (6) hineingezogen sowie aus ihm herausgeschoben werden kann,
und im zweiten Schlingenteil (3) der Draht nur in einem proximalen Bereich (5a) mit einer elektrischen Isolation versehen ist und in einem distalen Bereich (5b) ohne elektrische Isolation.

2. Resektionsschlinge nach Anspruch 1,
**dadurch gekennzeichnet, dass**
der Bereich ohne elektrische Isolation (5b) eine definierte Länge von 10 mm bis 30 mm hat.

3. Resektionsschlinge nach Anspruch 1 oder 2,
**dadurch gekennzeichnet, dass**
zumindest auf der elektrischen Isolation des ersten Schlingenteils (2) visuell erkennbare Markierungen (13a, 13b) vorhanden sind.

4. Resektionsschlinge nach Anspruch 3,
**dadurch gekennzeichnet, dass**
die Markierungen Ringe sind.

5. Resektionsschlinge nach Anspruch 4,
**dadurch gekennzeichnet, dass**
die Ringe mindestens zwei verschiedene Farben haben.

6. Resektionsschlinge nach Anspruch 3,
**dadurch gekennzeichnet, dass**
die Markierungen Zahlen zur Anzeige des Umfangs oder der Öffnungsweite der Schlinge sind.

7. Resektionsschlinge nach einem der vorhergehenden Ansprüche,
**dadurch gekennzeichnet, dass**
wenigstens eines der Schlingenteile (2, 3) federelastische, metallische Litze und/oder federelastischen, metallischen Rund- und/oder Flachdraht umfasst.

8. Resektionsschlinge nach einem der vorhergehenden Ansprüche,
**dadurch gekennzeichnet, dass**
die Schlinge an ihrem proximalen Ende (11) durch den Manipulationsdraht aus dem Katheter herausgeschoben und/oder in den Katheter hineingezogen werden kann.

9. Resektionsschlinge nach einem der Ansprüche 1 bis 8, **dadurch gekennzeichnet, dass** der Bereich ohne elektrische Isolation (5b) eine Antirutschbeschichtung oder kleine Zähne umfasst.

## Claims

1. RF surgical resection snare (1), comprising a snare consisting of a first snare section (2) and a second snare section (3), which form a snare tip (7) at their distal end (10), wherein both snare sections comprise a metallic wire and wherein in the first snare section the wire is completely electrically insulated,
wherein the snare is made in one piece,
**characterized in that**
the first snare section (2) and the second snare section (3) are connected at their proximal end (11) with a manipulation wire (8) being guided in a catheter (6), such that said snare can be pulled into the catheter (6) as well as be pushed out of the catheter (6) by means of said manipulation wire,
and in the second snare section (3), the wire is electrically insulated only in a proximal region (5a) and is electrically non-insulated in a distal region (5b).

2. Resection snare according to claim 1,
**characterized in that**
the region without electrical insulation (5b) has a defined length of 10 mm to 30 mm.

3. Resection snare according to claim 1 or 2,
**characterized in that**
visually recognizable markings (13a, 13b) are present at least on the electrical insulation of the first snare section (2).

4. Resection snare according to claim 3,
**characterized in that**
the markings are rings.

5. Resection snare according to claim 4,
**characterized in that**
the rings have at least two different colors.

6. Resection snare according to claim 3,
**characterized in that**
the markings are numbers which indicate the circumference or the opening width of the snare.

7. Resection snare according to one of the preceding claims,
**characterized in that**
at least one of the snare sections (2, 3) comprises spring-elastic, metallic strands and/or spring-elastic, metallic round and/or flat wire.

8. Resection snare according to one of the preceding claims,
**characterized in that**
the resection snare can be pushed out of the catheter and/or be pulled into the catheter by the manipulation wire at its proximal end (11).

9. Resection snare according to one of claims 1 to 8,
**characterized in that**
the electrically non-insulated region (5b) comprises an anti-slip coating or small teeth.

## Revendications

1. Boucle de résection (1) pour chirurgie HF, comprenant une boucle composée d'une première partie de boucle (2) et d'une deuxième partie de boucle (3) qui forment une pointe de boucle (7) à leur extrémité distale (10),
dans laquelle deux parties de boucle comprennent du fil métallique et le fil est muni d'une isolation électrique complète dans la première partie de boucle,
dans laquelle la boucle est réalisée d'une pièce,
**caractérisée en ce que**
la première partie de boucle (2) et la deuxième partie de boucle (3) sont reliées, à leur extrémité proximale (11), à un fil de manipulation (8) guidé dans un cathéter (6), de sorte que cette boucle peut être tirée à l'intérieur du cathéter (6) et poussée hors de celui-ci au moyen de ce fil de manipulation,
et le fil de la deuxième partie de boucle (3) est muni d'une isolation électrique seulement dans une région proximale (5a) et dépourvu d'isolation électrique dans une région distale (5b).

2. Boucle de résection selon la revendication 1, **caractérisée en ce que** la région dépourvue d'isolation électrique (5b) a une longueur définie de 10 mm à 30 mm.

3. Boucle de résection selon la revendication 1 ou 2, **caractérisée en ce que** des marquages reconnaissables visuellement (13a, 13b) sont présents au moins sur l'isolation électrique de la première partie de boucle (2).

4. Boucle de résection selon la revendication 3, **caractérisée en ce que** les marquages sont des bagues.

5. Boucle de résection selon la revendication 4, **caractérisée en ce que** les bagues ont au moins deux couleurs différentes.

6. Boucle de résection selon la revendication 3, **caractérisée en ce que** les marquages sont des nombres indiquant la circonférence ou la largeur d'ouverture de la boucle.

7. Boucle de résection selon l'une des revendications précédentes, **caractérisée en ce qu'**au moins une des parties de boucle (2, 3) comprend des torons métalliques élastiques comme des ressorts et/ou du fil métallique rond et/ou plat élastique comme un ressort.

8. Boucle de résection selon l'une des revendications précédentes, **caractérisée en ce que** la boucle peut être poussée hors du cathéter et/ou tirée dans le cathéter à son extrémité proximale (11) par le fil de manipulation.

9. Boucle de résection selon l'une des revendications 1 à 8, **caractérisée en ce que** la région dépourvue d'isolation électrique (5b) comprend un revêtement anti-dérapant ou de petites dents.
